# EUROPEAN PATENT SPECIFICATION

(11) **EP 1 337 243 B1**
(45) Date of publication and mention of the grant of the patent: **02.11.2006**
(21) Application number: 01980762.7
(22) Date of filing: 08.11.2001
(51) Int. Cl.: A61K 9/16, A61K 38/28, A61P 3/10

(54) **DELAYED-RELEASE PHARMACEUTICAL FORMULATIONS CONTAINING PROINSULIN C-PEPTIDE**
PROINSULIN C-PEPTID ENTHALTENDE ARZNEIZUBEREITUNG MIT VERZÖGERTER WIRKSTOFFFREIGABE
PREPARATIONS PHARMACEUTIQUES A ACTION RETARDEE CONTENANT DU PROINSULIN C-PEPTIDE

(30) Priority: 10.11.2000 DE 10055857
(43) Date of publication of application: 27.08.2003
(73) Proprietor: Creative Peptides Sweden AB, 182 64 Djursholm (SE)
(72) Inventor: MOHR, Detlef, 88400 Biberach (DE); SEIFFERT, Tim, 42697 Solingen (DE)
(74) Representative: Dzieglewska, Hanna Eva
(86) International application number: PCT/GB2001/004980
(87) International publication number: WO 2002/038129

(56) References cited:
- WO-A-02/15937
- WO-A-02/22211
- WO-A-02/38646
- WO-A-95/23175
- WO-A-98/13384
- US-A- 5 942 253
- JOHANSSON B L ET AL: "Beneficial effects of C-peptide on incipient nephropathy and neuropathy in patients with Type 1 diabetes mellitus." DIABETIC MEDICINE: A JOURNAL OF THE BRITISH DIABETIC ASSOCIATION. ENGLAND MAR 2000, vol. 17, no. 3, March 2000 (2000-03), pages 181-189, XP002207348 ISSN: 0742-3071 cited in the application

## Description

The invention relates to delayed-release pharmaceutical formulations containing proinsulin C-peptide.

Human proinsulin C-peptide which is formed when insulin is synthesised from proinsulin was regarded as biologically inactive for many years. However, very recently, various studies have shown that human proinsulin C-peptide may be pharmacologically effective in the treatment of diabetes and complications of diabetes such as peripheral neuropathy and nephthropathy (Johansson, Diabetic Medicine 17 (2000) 181-189; Wahren, J. Int. Med. 240 (1996) 115-124) and in the treatment of CNS disorders and for influencing the QT interval in the heart.

Because of the short half-life of plasma in humans, which is only about 40 minutes, C-peptide has to be administered continuously to the patient by subcutaneous long-term infusion (Forst, Exp Clin Diabetes 106 (1998) 270). However, this is hardly a possibility for diabetes sufferers who require constant C-peptide substitution.

C-peptide fragments or variants as described in International Application WO 98/13384 also have only a short biological half-life. WO 98/13384 discloses C-peptide fragments and their use in treating diabetes. Pharmaceutical compositions are described, including C-peptide covered by an envelope which may contain agents for prolonged release.

There was therefore a need for pharmaceutical formulations which contain proinsulin C-peptide and release the active substance continuously over a longer period

The problem underlying the invention was thus to provide a pharmaceutical formulation of C-peptide which is suitable for treating chronic diseases.

The problem was solved according to the invention by preparing a delayed-release pharmaceutical formulation which contains proinsulin C-peptide and which is exceptionally suitable for the long-term therapy of chronic diseases.

The terms "human proinsulin C-peptide" or "human C-peptide" in this patent application refer to a peptide with the sequence EADLQVGQVELGGGPGAGSLQPLALEGSLQ and pharmaceutically acceptable salts of the peptide.

The terms "proinsulin C-peptide" or "C-peptide" in this patent application refer to human proinsulin C-peptide or a biologically active analogue, derivative or fragment or pharmaceutically acceptable salt thereof.

The term "biologically active fragment of human proinsulin C-peptide" denotes a peptide fragment according to patent application WO 98/13384. In particular, the term relates to the peptide fragments EGSLQ, GSLQ, ELGGPGA, ELGG, ELGGP and GGPGA and peptides containing these peptide fragments.

The term "biologically active analogue of human C-peptide" denotes a peptide produced by conservative amino acid substitution from human C-peptide, while retaining the biological activity of human C-peptide. The biological activity of these C-peptide analogues can easily be measured by determining the Na⁺-K⁺-ATPase activity. A process for this determination is described in WO 98/13384. Another possible way of determining the biological activity of C-peptide is to measure the endothelial nitric oxide synthase (eNOS)-stimulating activity of C-peptide (Kunt *et al*, Exp. Clin Endocrinol Diabetes 106 (1998) 270).

The term "conservative amino exchange" denotes that one or more amino acids of human C-peptide are replaced by amino acids with a similar side chain. Families of amino acids with a similar side chain include, for example, amino acids with
- basic side chains (e.g. lysine, arginine, histidine)
- acid side chains (e.g. aspartic acid, glutamic acid)
- uncharged polar side chains (e.g. glycine, asparagine, glutamine, serine, threonine, tyrosine, cysteine)
- nonpolar side chains (e.g. alanine, valine, leucine, isoleucine, proline, phenylalanine, methionine, trytophan)
- branched side chains (e.g. threonine, valine, isoleucine) and
- aromatic side chains (e.g. tyrosine, phenylalanine, tryptophan, histidine).

Preferred analogues of human proinsulin-C-peptide are those wherein amino acids with small side chains are exchanged for one another, e.g. glycine for serine.

By the term "biologically active derivative of human C-peptide" is meant, in this patent application, a peptide which is obtained from human proinsulin C-peptide by the modification of a side chain while retaining the biological activity. Examples of this include modifications of the N-terminal amino group (e.g. by acetylation or by the addition of polyethyleneglycols), the carboxy-terminal group (e.g. by reduction, the introduction of an amino group or esterification) or one or more amino acid side groups (e.g. by hydroxylation, phosphorylation, acetylation, deamidation, reduction, oxidation, amination, halogenation, alkylation).

The term "pharmaceutically acceptable salts" denotes salts which retain the biological activity of C-peptide and do not produce any undesirable toxic effects. Examples of such salts are addition salts of inorganic or organic acids such as e.g. hydrochloric, phosphoric acid, acetic acid, tartaric acid, fumaric acid, malic acid, succinic acid or citric acid, salts of the anions thereof and salts of C-peptide with metal cations.

As discussed above in relation to the analogues of human C-peptide, convenient methods exist for determining the biological activity of these derivatives and salts. In all cases it will be appreciated that the analogues derivatives and salts may not have the same activity as C-peptide itself while still retaining a useful biological activity. For example an increase in half life may more than compensate for a modest reduction in absolute activity. The analogues, derivatives and salts will preferably have at least 50%, typically at least 70% of the biological activity of human proinsulin C-peptide.

The term "delayed-release pharmaceutical formulation" in this patent application denotes a pharmaceutical preparation from which the active substance is released in a therapeutically relevant amount under physiological conditions over a period of at least 24 hours. Preferred delayed-release formulations are those from which proinsulin C-peptide is released in therapeutically relevant amounts over a period of at least 5 days, preferably at least 10 days and most preferably at least 14 days. The word "delayed" does not imply that there must be an initial period of no release.

A therapeutically relevant "release" is conveniently measured *in vivo* by measuring the circulating plasma concentration of C-peptide. Any increase over the patient's basal circulating C-peptide plasma concentration (as measured prior to commencement of treatment) may be therapeutically relevant. However, preferably a "therapeutically relevant" C-peptide plasma concentration will exceed 0.5 ng/ml (0.15nM/ml), preferably it will exceed 0.7 ng/ml, e.g. about 1ng/ml (0.3nM/ml). In typical pharmacokinetic profiles, as shown by the Figures hereto, an initial burst in release of C-peptide is followed by a prolonged phase of sustained release.

In a preferred embodiment the delayed-release pharmaceutical formulation contains proinsulin C-peptide itself.

In another particularly preferred embodiment, the delayed-release pharmaceutical formulation contains an acetate salt of C-peptide. Pyroglutamate C-peptide is a further preferred active agent for inclusion in a delayed-release formulation.

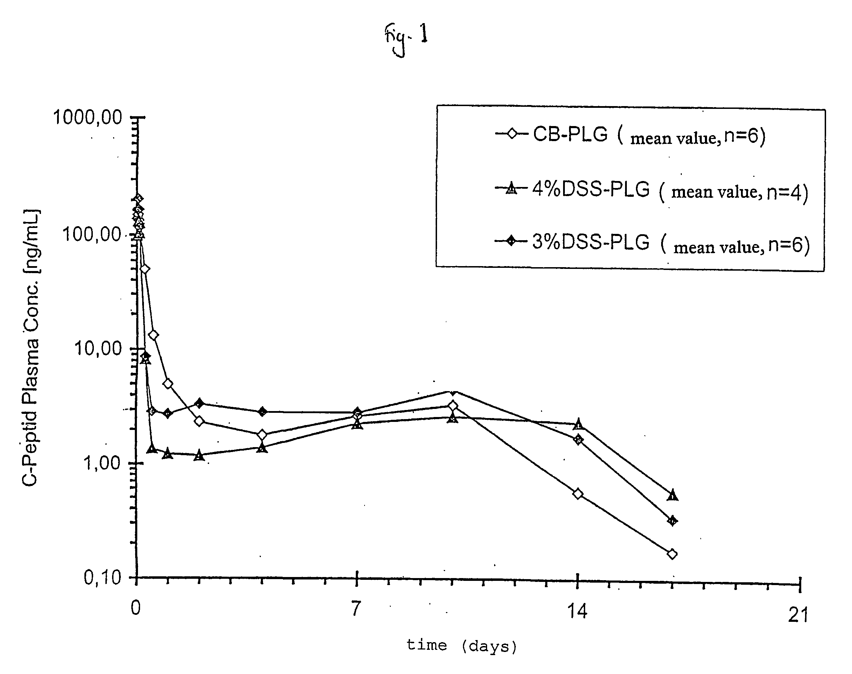

In order to delay the preferably parenterally administered C-peptide, the C-peptide was embedded in an absorbable matrix which significantly slows down the release of the peptide after administration to the patient and makes it possible to control the release of the C-peptide (cf. Figures 1-2).

The invention therefore relates to a delayed-release pharmaceutical formulation containing proinsulin C-peptide, characterised in that proinsulin C-peptide is present in an absorbable matrix comprising absorbable polymers, wherein said matrix comprises one or more polymers with glycolide and/or lactide units and wherein said matrix is obtainable by mixing lactide and glycolide units in the presence of a tin (II) catalyst and/or an electrolyte containing polyol which acts as a moderator without being incorporated in the polyester or bound thereto such that less than 1% of the polymer matrix is made up of the moderator under conditions which allow polymerisation thereof.

The invention also relates to a delayed-release pharmaceutical formulation containing proinsulin C-peptide, which is present in an absorbable matrix which comprises 2 or more poly lactide glycolide copolymer (PLG) polymers of different molecular weight.

Preferably, the absorbable matrix consists of absorbable polymers, or mixtures of absorbable polymers, in which the C-peptide is embedded in dispersed or dissolved form.

Polymers suitable for the preparation of delayed-release pharmaceutical formulations are known to those skilled in the art and include, for example, polylactide, polyglycolide, polylactide glycolide, polyanhydrides, polyorthoesters, polyacetals, polylactic acid, polyglycolic acid, polylactic acid polyglycolic acid, polycarbonates, polyether esters, cellulose derivatives, cyclodextrines, polyacrylates, polycaprolactones, polyvalerolactone, polypropiolactone, polybutyrolactone, polypivalolactone or polyester amides, of which polymers containing glycolide and/or lactide units, e.g. polylactide glycolide copolymers (PLG polymers), are preferred.

Particularly preferred matrix polymers are PLG polymers, especially those with a lactide/glycolide ratio of 75:25 to 25:75. D-,L- and/or D,L-lactide may be used.

Surprisingly, it has been found that the release of C-peptide from the delayed-release pharmaceutical formulations shows a particularly advantageous release profile if PLG polymers of two or preferably three different molecular weights are combined with one another.

In a preferred embodiment of the invention, the delayed-release pharmaceutical formulation therefore contains proinsulin C-peptide and an absorbable matrix, the absorbable matrix being made up of a mixture of at least two, preferably at least three different PLG polymers of different molecular weights.

Preferably, the polymer matrix contains
(A) 50-80% of a first polymer A with an average molecular weight of between 25 and 35 kDa
(B) 10-30% of a second polymer B with an average molecular weight of between 12 and 20 kDa (e.g. between 15 and 20 kDa)
(C) 10-20% of a third polymer C with an average molecular weight of between 1.5 and 3 kDa.

Preferably the formulations comprise 60-80% of a polymer of type (A), the remainder being made up of a mixture of one or more polymers of type (B) and (C). Polymers of type (A) and (B) conveniently have a lactide/glycolide molar ratio of about 50:50.

Most particularly preferred is a mixture of PLG polymers the polymer constituents of which are standardised for preparation in delayed-release pharmaceutical formulations. Examples include:
Polymer A: RG 503 or RG 503H (Boehringer Ingelheim)
Polymer B: RG 502 or RG 502H (Boehringer Ingelheim)
Polymer C: Mn 2300 (Boehringer Ingelheim).

In another aspect of the invention, the absorbable matrix of the delayed-release formulation according to the invention contains polymers prepared by ring-opening polymerisation of lactide and/or glycolide units. These two species are preferably mixed at a ratio of 75:25 to 25:75, preferably 60:40 to 40:60.

Surprisingly, it was found that the polymerisation of lactide and/or glycolide in an extruder, especially in the presence of large amounts of catalyst of more than 1000 ppm of tin (II) and/or in the presence of electrolyte-containing polyols as moderators, e.g. a suitably charge balanced dextran sulphate such as dextran sodium sulphate (DSS), produces PLG polymers which have particularly advantageous release profiles for C-peptide.

The invention therefore provides a delayed-release pharmaceutical formulation containing C-peptide and an absorbable polymer matrix, the polymer matrix being comprising a PLG polymer produced by ring-opening polymerisation in an extruder. Preferably the polymer matrix consists essentially of one or more PLG polymers.

In particularly preferred embodiments polymerisation was carried out in the extruder using at least 1000 ppm of tin(II) based on the amount of lactide/glycolide put in.

In another preferred embodiment, polymerisation was carried out in the extruder in the presence of a moderator, most preferably in the presence of dextran sodium sulphate (DSS). Preferably 1 to 5 wt.% (e.g. 2 to 4%) of DSS is added to the prepolymer mix.

Alternatively viewed, the present invention provides a delayed-release pharmaceutical formulation containing proinsulin C-peptide and an absorbable polymer matrix obtainable by mixing lactide and glycolide units under conditions which allow polymerisation thereof. Preferably the units are mixed in an extruder and ring-opening polymerisation takes place. Preferably polymerisation takes place in the presence of at least 1000 ppm of a tin (II) catalyst (e.g. tin(II)2-ethylhexanoate) and/or an electrolyte-containing polyol (e.g. DSS) which acts as a moderator.

By "moderator" in this patent application is meant a substance which is added before the polymerisation process when preparing an absorbable polyester and which influences the molecular weight, the breakdown rate and the release properties of corresponding formulations without acting as an independent polymerisation catalyst and without being incorporated in the polyester or bound thereto in any substantial amount. Typically less than 1% of the polymer matrix is made up of the moderator, preferably <0.5%, e.g. 0.001 to 0.2% of the polymer matrix is moderator.

With the PLG polymers it has been possible to prepare an injectable delayed-release form of proinsulin C-peptide which allows the proinsulin C-peptide to be released in therapeutically effective (relevant) amounts over a period of several days. At the same time, it is released surprisingly uniformly , which is ideally suited to the delayed-release administration of proinsulin C-peptide. A burst in the release of proinsulin C-peptide may be observed for the first day or two after administration, thereafter release (as reflected by circulating plasma levels) is surprisingly uniform.

The invention therefore relates to the use of proinsulin C-peptide, particularly human proinsulin C-peptide, in the manufacture of a delayed-release pharmaceutical formulation. The proinsulin C-peptide is released in therapeutically relevant amounts from a delayed-release pharmaceutical formulation of this kind according to the invention over at least 1 day, 5 days, 10 days or, preferably, at least 14 or 21 days, for example.

The delayed-release pharmaceutical formulations containing proinsulin C-peptide according to the invention may be in the form of implants, films, patches, pellets, granules, microcapsules or microparticles.

Delayed-release pharmaceutical formulations which contain microparticles containing proinsulin C-peptide and an absorbable matrix are preferred.

The preparation of delayed-release preparations of this kind is known *per se* in the art.

However, when preparing formulations containing human proinsulin C-peptide, it was surprisingly found that owing to the strong water-solubility of human C-peptide, methods of preparation based on phase separations lead to unacceptably high losses of active substance. Examples of less suitable processes of this kind connected with phase separations are multiple emulsion processes, e.g. W/O/W or W/O/O processes.

Therefore, methods of preparing the delayed-release preparations in which the charging of the polymers with active substance is carried out in single-phase systems or wherein the solvent phase is evaporated off are preferred. Examples of suitable methods include the preparation of implants, films or pellets by C-peptide charging of the polymers in extruders or the preparation of particles of active substance by ASES methods as described in European Patent EP 563 176.

Particularly preferably, the delayed-release forms according to the invention are prepared by processing polymer/proinsulin C-peptide mixtures by spray drying to form microparticles. For this, the polymer mixture is dissolved in a solvent, the polypeptide is also dissolved and stirred into the polymer solution. Finally, the solution is sprayed until the polymer/polypeptide mixture is precipitated in particle form.

The invention therefore relates to microparticles consisting of an absorbable polymer matrix in which proinsulin C-peptide, most preferably human proinsulin C-peptide, is dispersed, dissolved or suspended. Microparticles measuring not more than 10 µm are preferred.

The invention also relates to delayed-release pharmaceutical formulations comprising microparticles which contain proinsulin C-peptide and an absorbable matrix, the microparticles being prepared by spray drying.

The delayed-release formulations according to the invention may contain a proportion of proinsulin C-peptide of 1-50% (w/w) based on the dry content, while a proinsulin C-peptide content of 1-30% (w/w) and most particularly 3-15% (w/w) is preferred.

The proportion of the absorbable matrix in the delayed-release formulation according to the invention is between 5 and 99% (w/w) of the dry mass, preferably between 50 and 97% (w/w) and most preferably between 80 and 96% (w/w).

In a typical example the dry formulation consists of
90% of absorbable polymers and
10% of human proinsulin C-peptide.

This dry formulation can be taken up before use in a pharmaceutically acceptable solvent which also contains other ingredients which are well known to those skilled in the art of parenteral drug preparations. Examples are buffers, electrolytes, stabilisers, preservatives and/or detergents.

Alternatively, the pharmaceutical excipients may already be present in the dry formulation, which may then have the following composition, for example:

| | |
|---|---|
| 80% | polymer |
| 7% | proinsulin C-peptide |
| 3.25% | NaCl |
| 0.5% | detergent |
| 5.75% | cellulose derivative |
| 0.5% | preservative |
| 3% | buffer |

A formulation of this kind would preferably be reconstituted before use by taking it up in electrolyte-free solvent, e.g. in pyrogen-free water.

The pharmaceutical formulation ready for use then contains, for example:
277.5 mg of polymer
22.5 mg of human proinsulin C-peptide
13 mg of NaCl
1.5 mg of Tween
12 mg of carboxymethylcellulose
1.5 ml of water

The polymer and the proinsulin C-peptide preferably being in the form of microparticles and the proinsulin C-peptide preferably being dissolved or dispersed in the polymer matrix.

The invention further relates to a kit comprising the delayed-release pharmaceutical formulation of microparticles of the invention and a device for administering said formulation or microparticles.

The invention also relates to the use of the pharmaceutical formulations of proinsulin C-peptide according to the invention for treating diabetes and complications of diabetes, particularly diabetic neuropathy, diabetic nephthropathy, diabetic retinopathy or to reduce the electrocardiographic QT interval.

The delayed-release formulations according to the invention are intended particularly for parenteral administration. Preferred methods of administration are by subcutaneous, intramuscular and intracutaneous route.

Subcutaneous administration is particularly preferred.

The pharmaceutical formulation according to the invention may contain, in addition to proinsulin C-peptide, another pharmacologically active substance which is suitable for the prevention, treatment or alleviation of diabetes or complications of diabetes.

Useful examples of these are combinations with insulin, insulin derivatives or insulin-like growth factor, antidiabetics, painkillers, neurotrophines such as Nerve Growth Factor, or ACE inhibitors. These additional active substances may be present in the same formulations or in separate formulations, e.g. as a kit-of-parts, for simultaneous, separate or sequential administration.

The examples which follow serve to illustrate the invention in more detail and may be read with reference to the figures in which:
Figure 1 shows the mean values of *in vivo* release profiles (in beagles) of microparticles each charged with 7-7.5% proinsulin C-peptide and the matrix of which consists of a polymer mixture consisting of PLG polymer RG 503 H + PLG polymer RG 502 H + Mn 2300 in the ratio 80/10/10 ("Commercial Blend", CB-PLG) or a PLG polymer prepared by ring-opening polymerisation of lactide and glycolide with the addition of 3% dextrane sodium sulphate (DSS) or 4% DSS in the extruder (DSS-PLG).

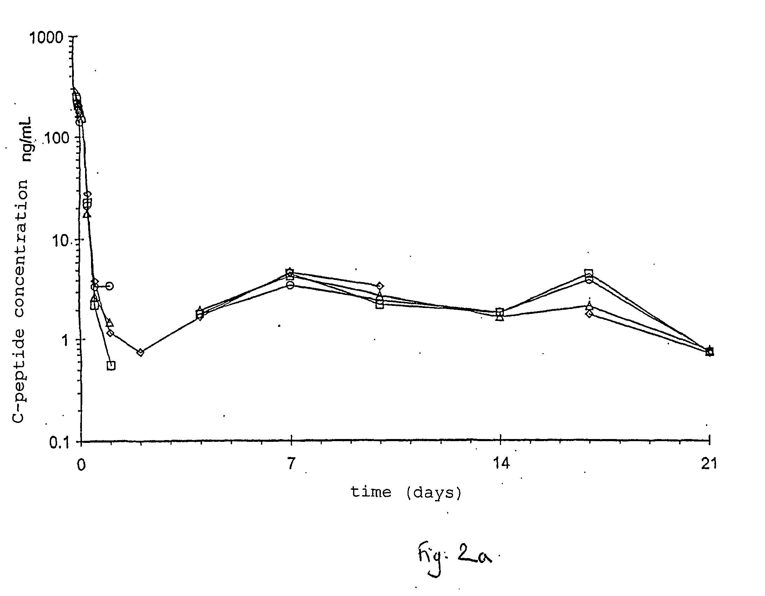

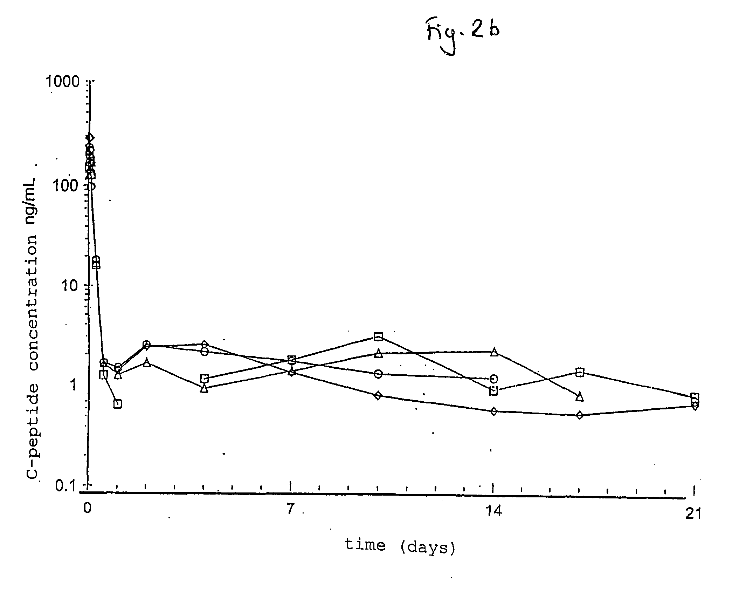

Figure 2 shows individual *in vivo* release profiles (in beagles) of microparticles each charged with 7.5% C-peptide and the matrix of which consists of DSS-modified PLG polymers. Figure 2a shows four release profiles of 3% DSS-modified polyesters which were produced using 1200 ppm of Sn, Figure 2b shows release profiles of 3% DSS-modified polyesters produced using 1600 ppm of Sn.

### Example 1: Preparation of polymers in an extruder

### A. Polymerisation:

First, the DSS is dried for at least 3 days at ambient temperature in a fine vacuum (about 0.1 mbar).

53 mol-% of D,L-lactide, 47 mol-% of glycolide and the predried DSS (2 to 4 wt.%) are weighed into a mixing vessel. To produce a homogeneous mixture the ingredients are mixed for 30 min in a gyrowheel mixer.

The monomer/DSS premix is poured into a metering balance of the extruder (Leistritz double screw extruder type LSM 34 GG) and then metered continuously into the extruder by means of metering screws.

A defined amount of the catalyst tin(II)2-ethylhexanoate is dissolved in toluene and this solution is then continuously metered into the extruder by means of an HPLC pump. The delivery rate is adjusted, taking into account the feed rate, so that the quantity of catalyst in the reaction mixture is 1200 ppm.

The polymerisation is then carried out in the extruder at temperatures between 170 and 220°C.

### B. Working up

The crude polymer is dissolved in acetone overnight. After it has dissolved completely, D,L-lactic acid is added and stirring is continued for another 2 to 3 hours. The cloudy solution obtained (10 wt.% of polyester in solvent mixture) is filtered off under a slight nitrogen pressure.

The filtered polyester solution is precipitated with demineralised water. The polyester precipitated is washed, filtered and then dried at a maximum of (35°C) until a constant weight is achieved.

### Example 2: Preparation of polymer mixtures

The following commercially available (Boehringer Ingelheim) polymers were mixed before being formulated by spray drying as described in Example 3 below. In each case, amounts given are in % wt/wt.

| | | |
|---|---|---|
| (a) | RG 503H : | 80 |
| | RG 502H : | 10 |
| | Mn 2300 : | 10 |
| | | |
| (b) | RG 503H : | 70 |
| | RG 502H : | 20 |
| | Mn 2300 : | 10 |
| | | |
| (c) | RG 503H : | 60 |
| | RG 502H : | 20 |
| | Mn 2300 : | 20 |

The release profile for formulation (a) is shown in Fig. 1

### Example 3: Description of the preparation of microparticles

0.55 g of polymer, or polymer dry mix, was dissolved in 6 ml of glacial acetic acid, 0.045 g of human C-peptide (C-peptide acetate/Polypeptide Laboratories, Wolfenbüttel, Germany; Cat. No. P-0764) was dissolved in 0.15 ml of water and 3.0 ml of glacial acetic acid and slowly dissolved in the polymer solution. The solution is sprayed at 60°C in a Büchi 190 spray drier at 60°C and dried until the microparticles can be obtained as a fine flowing powder.

This method of microparticle production may be used in respect of the polymers produced according to Example 1 or 2.

### Example 4: Delayed-release pharmaceutical formulation:

### (A) Dry formulation:

300 mg of microparticles containing 277.5 mg of polymer and 22.5 mg of human proinsulin-C-peptide.

### (B) Reconstitution of the dry formulation to produce the delayed-release preparation ready for use

300 mg of microparticles containing 22.5 mg of human C-peptide are resuspended in 1.5 ml of solvent (0.9% common salt, 0.1% Tween, 0.8% sodium carboxymethylcellulose).

### Example 5: in vivo release rate of C-peptide from microparticles

Beagles were subcutaneously given 300 mg of microparticles loaded with 7.5% of human C-peptide. At the specified times, blood was taken from the animals and the C-peptide plasma levels were quantified using LC-MS. The results are shown in Figures 1 and 2.

## Claims

1. A delayed-release pharmaceutical formulation containing proinsulin C-peptide which is present in an absorbable matrix comprising absorbable polymers, wherein said matrix comprises one or more polymers with glycolide and/or lactide units and wherein said matrix is obtainable by mixing lactide and glycolide units in the presence of a tin (II) catalyst and/or an electrolyte containing polyol which acts as a moderator without being incorporated in the polyester or bound thereto such that less than 1% of the polymer matrix is made up of the moderator under conditions which allow polymerisation thereof.

2. A formulation according to claim 1 wherein said polyol which acts as a moderator is dextran sodium sulphate.

3. A delayed-release pharmaceutical formulation containing proinsulin C-peptide, which is present in an absorbable matrix which comprises 2 or more poly lactide glycolide copolymer (PLG) polymers of different molecular weight.

4. A formulation according to claim 3 which comprises 3 PLG polymers of different molecular weights.

5. A formulation according to claim 4 which comprises a first polymer with an average molecular weight of between 25 and 35 kDA, a second polymer with an average molecular weight of between 12 and 20 kDA and a third polymer with an average molecular weight of between 1.5 and 3 kDA.

6. A formulation according to claim 5 which comprises 50-80% by weight of said first polymer, 10-30% by weight of said second polymer and 10-20% by weight of said third polymer.

7. A formulation according to one any of the preceding claims, wherein the proinsulin C-peptide is present in a concentration of 1 to 30% (w/w) based on the dry content of the delayed-release formulation.

8. A formulation according to any one of the preceding claims, wherein the delayed-release pharmaceutical formulation comprises microparticles which contain proinsulin C-peptide and an absorbable matrix.

9. A formulation according to claim 8, wherein the microparticles are produced by spray drying.

10. A formulation according to any one of the preceding claims for parenteral administration.

11. A formulation according to any one of the preceding claims for subcutaneous, intracutaneous or intramuscular administration.

12. A formulation according to any one of the preceding claims which provides release, in vivo, of therapeutically relevant amounts of proinsulin C-peptide for at least 5 days.

13. A formulation according to claim 12 which provides release, *in vivo,* of therapeutically relevant amounts of proinsulin C-peptide for at least 10 days.

14. A formulation according to any one of the preceding claims for treating diabetes or complications of diabetes or for shortening the electrocardiographic QT interval.

15. A formulation according to any one of the preceding claims for treating diabetic neuropathy, diabetic nephthropathy or diabetic retinopathy.

16. A formulation according to one of the preceding claims further comprising another active substance for the prevention, treatment or alleviation of diabetes or complications of diabetes.

17. A formulation, according to any one of the preceding claims, wherein the proinsulin C-peptide is human C-peptide.

18. A formulation, according to any one of the preceding claims, wherein the proinsulin C-peptide is in the form of an acetate salt.

19. A kit comprising a delayed-release pharmaceutical formulation or microparticles according to one of the preceding claims and a device for administering said formulation or microparticles.

20. A method of preparing a pharmaceutical formulation for delayed-release of proinsulin C-peptide which comprises mixing lactide and glycolide units in the presence of a tin (II) catalyst and/or an electrolyte containing polyol, which acts as a moderator, without being incorporated in the polyester or bound thereto, such that less than 1% of the polymer matrix is made up of the moderator, under conditions which allow polymerisation thereof and combining the polymer mix obtained thereby with proinsulin C-peptide.

21. A method according to claim 20 wherein said polyol is dextran sodium sulphate.

22. A method of preparing a pharmaceutical formulation for delayed-release of proinsulin C-peptide which comprises mixing 2 or more PLG polymers as defined in claims 5 or 6 to form a polymer mix and mixing simultaneously or sequentially combining the polymer mix with proinsulin C-peptide.

23. A method as claimed in any one of claims 20 to 22 wherein the polymer mix and proinsulin C-peptide are mixed by spray drying to form microparticles.

24. A method as claimed in claim 23 wherein the polymer mix and proinsulin C-peptide are dissolved in solvents and a solvent mixture of both the polymer mix and proinsulin C-peptide is sprayed such that the polymer/peptide mixture is precipitated in particulate form.

## Patentansprüche

1. Pharmazeutische Zubereitung zur verzögerten Wirkstoffabgabe mit Proinsulin C-Peptid in einer resorbierbaren Matrix, die resorbierbare Polymere aufweist, wobei die besagte Matrix ein oder mehrere Polymere mit Glykolid- und/oder Laktid-Einheiten umfasst und wobei die besagte Matrix erhältlich ist durch Vermischen der Laktid- und Glykolid-Einheiten in Anwesenheit eines Zinn-(II)-Katalysators und/oder eines einen Elektrolyten enthaltenden Polyols, das als ein Moderator wirkt, ohne in das Polyester eingebettet oder an dieses gebunden zu sein, so dass weniger als 1% der Polymermatrix aus dem Moderator unter Bedingungen gefertigt ist, die deren Polymerisation ermöglichen.

2. Zubereitung gemäß Anspruch 1, wobei das besagte als ein Moderator wirkende Polyol Dextran-Natriumsulfat ist.

3. Pharmazeutische Zubereitung zur verzögerten Wirkstoffabgabe mit Proinsulin C-Peptid in einer resorbierbaren Matrix, die zwei oder mehrere Polylaktid-Glykolid-Copolymer-(PLG)-Polymere mit unterschiedlichem Molekulargewicht aufweist.

4. Zubereitung gemäß Anspruch 3, die drei PLG-Polymere mit unterschiedlichen Molekulargewichten aufweist.

5. Zubereitung gemäß Anspruch 4, die ein erstes Polymer mit einem mittleren Molekulargewicht zwischen 25 und 35 kDA, ein zweites Polymer mit einem mittleren Molekulargewicht zwischen 12 und 20 kDA und ein drittes Polymer mit einem mittleren Molekulargewicht zwischen 1,5 und 3 kDA aufweist.

6. Zubereitung gemäß Anspruch 5, die 50-80 Gew.-% des besagten ersten Polymers, 10-30 Gew.-% des besagten zweiten Polymers und 10-20 Gew.-% des besagten dritten Polymers aufweist.

7. Zubereitung gemäß einem der vorhergehenden Ansprüche, wobei das Proinsulin C-Peptid in einer Konzentration von 1 bis 30% (Gew./Gew.) bezogen auf den Trockenanteil der Zubereitung zur verzögerten Wirkstoffabgabe vorliegt.

8. Zubereitung gemäß einem der vorhergehenden Ansprüche, wobei die Zubereitung zur verzögerten Wirkstoffabgabe Mikropartikel umfasst, die Proinsulin C-Peptid und eine resorbierbare Matrix enthalten.

9. Zubereitung gemäß Anspruch 8, wobei die Mikropartikel durch Sprühtrocknen hergestellt sind.

10. Zubereitung gemäß einem der vorhergehenden Ansprüche zur parenteralen Verabreichung.

11. Zubereitung gemäß einem der vorhergehenden Ansprüche zur subkutanen, intrakutanen oder intramuskulären Verabreichung.

12. Zubereitung gemäß einem der vorhergehenden Ansprüche, die in vivo eine Abgabe von therapeutisch bedeutsamen Mengen an Proinsulin C-Peptid über wenigstens 5 Tage hinweg bereitstellt.

13. Zubereitung gemäß Anspruch 12, die in vivo eine Abgabe von therapeutisch bedeutsamen Mengen an Proinsulin C-Peptid über wenigstens 10 Tage hinweg bereitstellt.

14. Zubereitung gemäß einem der vorhergehenden Ansprüche zur Behandlung von Diabetes oder von Komplikationen der Diabetes oder zum Verkürzen des elektrokardiographischen QT-Intervalls.

15. Zubereitung gemäß einem der vorhergehenden Ansprüche zur Behandlung der diabetischen Neuropathie, der diabetischen Nephropathie oder der diabetischen Retinopathie.

16. Zubereitung gemäß einem der vorhergehenden Ansprüche, die weiterhin eine andere aktive Substanz zur Vorbeugung, zur Behandlung oder zur Linderung von Diabetes oder von Komplikationen von Diabetes aufweist.

17. Zubereitung gemäß einem der vorhergehenden Ansprüche, wobei das Proinsulin C-Peptid menschliches C-Peptid ist.

18. Zubereitung gemäß einem der vorhergehenden Ansprüche, wobei das Proinsulin C-Peptid in Gestalt eines Acetatsalzes vorliegt.

19. Einrichtung, die eine pharmazeutische Zubereitung oder Mikropartikel zur verzögerten Wirkstoffabgabe gemäß einem der vorhergehenden Ansprüche und eine Vorrichtung zur Verabreichung der besagten Zubereitung oder der Mikropartikel aufweist.

20. Verfahren zum Herstellen einer pharmazeutischen Zubereitung zur verzögerten Abgabe von Proinsulin C-Peptid, welches das Vermischen von Laktat- und Glykolideinheiten in Anwesenheit eines Zinn-(II)-Katalysators und/oder eines einen Elektrolyten enthaltenden Polyols, das als ein Moderator wirkt, ohne in das Polyester eingebettet oder an dieses gebunden zu sein, so dass weniger als 1% der Polymermatrix aus dem Moderator gefertigt ist, unter Bedingungen, die deren Polymerisation ermöglichen, und das Kombinieren der auf diese Weise erhaltenen Polymermischung mit Proinsulin C-Peptid umfasst.

21. Verfahren nach Anspruch 20, wobei das besagte Polyol Dextran-Natriumsulfat ist.

22. Verfahren zum Herstellen einer pharmazeutischen Zubereitung zur verzögerten Abgabe von Proinsulin C-Peptid, welches das Vermischen von zwei oder mehreren PLG-Polymeren gemäß den Ansprüchen 5 oder 6 zum Ausbilden einer Polymermischung und das gleichzeitige Vermischen oder das aufeinander folgende Kombinieren der Polymermischung mit Proinsulin C-Peptid umfasst.

23. Verfahren nach einem der Ansprüche 20 bis 22, wobei die Polymermischung und das Proinsulin C-Peptid durch Sprühtrocknen unter Ausbildung von Mikropartikeln vermischt werden.

24. Verfahren nach Anspruch 23, wobei die Polymermischung und das Proinsulin C-Peptid in Lösungsmitteln gelöst werden, wobei eine die Polymermischung und das Proinsulin C-Peptid enthaltende Lösungsmittelmischung so versprüht wird, dass die Polymer/Peptid-Mischung in Partikelgestalt ausgefällt wird.

## Revendications

1. Composition pharmaceutique à libération retardée comprenant le peptide C de pro-insuline qui est présent dans une matrice absorbable comprenant des polymères absorbables, dans laquelle ladite matrice comprend un ou plusieurs polymères avec des motifs glycolide et/ou lactide et dans laquelle ladite matrice peut être obtenue par mélange de motifs lactide et glycolide en présence d'un catalyseur à l'étain (II) et/ou d'un polyol contenant un électrolyte qui agit comme un modérateur sans être incorporé dans le polyester ou lié à celui-ci de façon que moins de 1 % de la matrice polymère soit constitué du modérateur dans des conditions qui permettent sa polymérisation.

2. Formulation selon la revendication 1, dans laquelle ledit polyol qui agit comme un modérateur est le sulfate de dextrane sodique.

3. Composition pharmaceutique à libération retardée contenant le peptide C de pro-insuline, qui est présent dans une matrice absorbable comprenant 2 polymères de copolymère de poly(lactide-glycolide) (PLG) ou plus ayant des masses moléculaires différentes.

4. Formulation selon la revendication 3, qui comprend 3 polymères de PLG ayant des masses moléculaires différentes.

5. Formulation selon la revendication 4, qui comprend un premier polymère ayant une masse moléculaire moyenne comprise entre 25 et 35 kDa, un deuxième polymère ayant une masse moléculaire moyenne comprise entre 12 et 20 kDa et un troisième polymère ayant une masse moléculaire moyenne comprise entre 1,5 et 3 kDa.

6. Formulation selon la revendication 5, qui comprend 50 à 80 % en poids dudit premier polymère, 10 à 30 % en poids dudit deuxième polymère et 10 à 20 % en poids dudit troisième polymère.

7. Formulation selon l'une quelconque des revendications précédentes, dans laquelle le peptide C de pro-insuline est présent à une concentration de 1 à 30 % (en poids/poids) par rapport à la teneur en extrait sec de la formulation à libération retardée.

8. Formulation selon l'une quelconque des revendications précédentes, dans laquelle la formulation pharmaceutique à libération retardée comprend des microparticules qui contiennent le peptide C de pro-insuline et une matrice absorbable.

9. Formulation selon la revendication 8, dans laquelle les microparticules sont produites par séchage par pulvérisation.

10. Formulation selon l'une quelconque des revendications précédentes, pour administration par voie parentérale.

11. Formulation selon l'une quelconque des revendications précédentes, pour administration par voie sous-cutanée, intracutanée ou intramusculaire.

12. Formulation selon l'une quelconque des revendications précédentes, qui réalise la libération in vivo de quantités thérapeutiques pertinentes de peptide C de pro-insuline pendant au moins 5 jours.

13. Formulation selon la revendication 12, qui réalise la libération in vivo de quantités thérapeutiques pertinentes de peptide C de pro-insuline pendant au moins 10 jours.

14. Formulation selon l'une quelconque des revendications précédentes, pour traiter le diabète ou des complications du diabète ou pour raccourcit l'intervalle QT électrocardiographique.

15. Formulation selon l'une quelconque des revendications précédentes, pour traiter la neuropathie diabétique, la néphropathie diabétique ou la rétinopathie diabétique.

16. Formulation selon l'une quelconque des revendications précédentes, comprenant en outre une autre substance active pour la prévention, le traitement ou le soulagement du diabète ou de complications du diabète.

17. Formulation selon l'une quelconque des revendications précédentes, dans laquelle le peptide C de pro-insuline est le peptide C humain.

18. Formulation selon l'une quelconque des revendications précédentes, dans laquelle le peptide C de pro-insuline est sous la forme d'un sel acétate.

19. Trousse comprenant une formulation pharmaceutique à libération retardée ou des microparticules selon l'une des revendications précédentes et un dispositif pour administrer ladite formulation ou lesdites microparticules.

20. Procédé pour préparer une formulation pharmaceutique pour la libération retardée de peptide C de pro-insuline, qui comprend le mélange de motifs lactide et glycolide en présence d'un catalyseur à l'étain (II) et/ou d'un polyol contenant un électrolyte, qui agit comme un modérateur sans être incorporé dans le polyester ou lié à celui-ci de façon que moins de 1 % de la matrice polymère soit constitué du modérateur, dans des conditions qui permettent sa polymérisation, et la combinaison du mélange de polymères ainsi obtenu avec le peptide C de pro-insuline.

21. Procédé selon la revendication 20, dans lequel ledit polyol est le sulfate de dextrane sodique.

22. Procédé pour préparer une formulation pharmaceutique pour la libération retardée de peptide C de pro-insuline, qui comprend le mélange de 2 polymères de PLG ou plus, tels que définis dans la revendication 5 ou 6, pour former un mélange de polymères, et le mélange simultané ou la combinaison séquentielle du mélange de polymères avec le peptide C de pro-insuline.

23. Procédé selon l'une quelconque des revendications 20 à 22, dans lequel le mélange de polymères et le peptide C de pro-insuline sont mélangés par séchage par pulvérisation pour former des microparticules.

24. Procédé selon la revendication 23, dans lequel le mélange de polymères et le peptide C de pro-insuline sont dissous dans des solvants et un mélange solvant à la fois du mélange de polymères et du peptide C de pro-insuline est pulvérisé de façon que le mélange de polymères/peptide soit précipité sous forme particulaire.
